# EUROPEAN PATENT APPLICATION

(11) **EP 3 560 541 A1**
(43) Date of publication of application: **30.10.2019**
(21) Application number: 19180906.0
(22) Date of filing: 19.12.2014
(51) Int. Cl.: A61M 25/06, A61M 25/00, A61M 5/158, A61M 5/32

(54) **SAFETY IV CATHETER ASSEMBLY**

(30) Priority: 20.12.2013 US 201361918939 P
(62) Divisional of application: 14821635.1
(71) Applicant: B. Braun Melsungen AG, 34212 Melsungen (DE)
(72) Inventor: TEOH, Teng Sun, 11400 Air Itam, Penang (MY)
(74) Representative: Kinkeldey, Daniela

(57) **Abstract**

A safety IV catheter assembly (100) comprising: a catheter hub (104) with a catheter tube (102) extending distally from the catheter hub; a needle hub (108) proximal of the catheter hub having a needle (106) extending distally from the needle hub and through the catheter hub and the catheter tube, the needle including a sharp distal tip (112) extending distally of a distal end of the catheter tube; and a tip protector assembly (116) located on the needle between the catheter hub and the needle hub or completely inside the catheter hub, the tip protector assembly including: a proximal wall (174), an opening (176) in the proximal wall accommodating the needle, first and second fingers (168, 176; 206, 208) extending distally from the proximal wall; and a retaining clip (138) extending around distal portions of the first and second fingers or a resilient member (216) extending through both the first and second fingers; wherein the first finger includes a tab (184; 210) extending radially inward and bearing against the needle to deflect the first finger radially outward against a spring restoring force of the retaining clip or the resilient member in a ready to use position; and wherein the retaining clip or the resilient member increases the spring restoring force tending to press the first and second fingers radially inward.

## Description

### FIELD OF ART

The present invention is generally directed to needle safety assemblies and related methods and more particularly to needle safety assemblies and related methods utilizing needle guards.

### BACKGROUND

Insertion procedure for an intravenous (IV) catheter assembly contains four basic steps: (1) the healthcare worker inserts the needle and catheter together into the patient's vein; (2) after insertion into the vein with the needle point, the catheter is forwarded into the vein of the patient by the healthcare worker pushing the catheter with his or her finger; (3) the healthcare worker withdraws the needle by grasping the hub end (opposite the point end) while at the same time applying pressure to the patient's skin at the insertion site with his or her free hand to stop the flow of blood through the catheter; and (4) the healthcare worker then tapes the exposed end of the catheter (the catheter hub) to the patient's skin and connects it to the source of the fluid to be administered into the patient's vein.

One problem is that, immediately after the withdrawal of the needle from the patient's vein, the healthcare worker, who is at this time involved in at least two urgent procedures, must place the exposed needle tip at a nearby location and address the tasks required to accomplish the needle withdrawal. It is at this juncture that the exposed needle tip creates a danger of an accidental needle stick, which, under the circumstances, leaves the healthcare worker vulnerable to the transmission of various dangerous blood-borne pathogens, including AIDS and hepatitis.

Other needle types similarly expose healthcare workers to risks of accidental needle sticks. For example, a doctor administering an injection, using a straight needle, a Huber needle, an epidural needle, etc., may place the used needle on a tray for subsequent disposal by a nurse. For the period between placing the used needle on a tray or a work station to the time it is discarded, the used needle is a potential source for disease transmissions for those that work near or around the needle.

### SUMMARY

The various embodiments of a needle assembly have several features, no single one of which is solely responsible for their desirable attributes. Without limiting the scope of the present embodiments as set forth in the claims that follow, their more prominent features now will be discussed briefly.

Aspects of the present disclosure include a safety needle assembly that includes a needle having a sharp distal tip and a needle tip protector mounted on the needle. The needle tip protector includes a first portion having a channel receiving the needle and a second portion attached to the first portion. The second portion has a proximal wall, an opening in the proximal wall receiving the needle, a sidewall extending distally from the proximal wall, first and second spring arms extending transversely from opposite edges of the sidewall, and a third spring arm extending distally from the sidewall. The first spring arm and the second spring arm extend at least partially around the first portion. The third spring arm includes a tab extending radially inward and configured to bear against the needle to deflect the third spring arm radially outward against a spring restoring force in a ready to use position.

The first spring arm and the second spring arm can be arcuate. The first spring arm and the second spring arm can be deflected outwardly by the first portion against a spring restoring force to secure the second portion to the first portion.

The first portion can have a mating surface having a depth configured to receive the first spring arm and the second spring arm.

The tab of the third spring arm can have an inverted V-shaped profile defining a space in between the tab to receive the sharp distal tip.

The needle can have a change in profile proximal of the sharp distal tip, the change in profile being larger than the opening of the proximal wall of the second portion.

The first portion and the second portion can each define a shoulder configured to engage with a catheter hub to retain the needle tip protector to the catheter hub in the ready to use position, and disengage with the catheter hub when the third spring arm and the tab elastically return to cover the sharp distal tip.

The second portion can further include a strengthening rib at a junction of the proximal wall and the sidewall for increasing the spring restoring force of the third spring arm.

Another aspect of the present disclosure includes a safety IV catheter assembly that includes a catheter hub, a catheter tube extending distally from the catheter hub, a needle hub proximal of the catheter hub, a needle extending distally from the needle hub and through the catheter hub and the catheter tube, the needle including a sharp distal tip, and a tip protector assembly located on the needle between the catheter hub and the needle hub. The tip protector assembly includes a first portion having a channel for receiving the needle and a second portion attached to the first portion. The second portion has a proximal wall, an opening in the proximal wall for accommodating the needle, a spring arm extending distally from a first edge of the proximal wall, and a retaining arm extending distally from a second edge of the proximal wall. The spring arm includes a tab extending radially inward and bearing against the needle to deflect the spring arm radially outward against a spring restoring force. The retaining arm includes a first retaining feature engaging a second retaining feature on the catheter hub to releasably secure the tip protector assembly to the catheter hub.

The first retaining feature can include a protrusion extending radially inward from the retaining arm. The second retaining feature comprises a groove to receive the protrusion.

The tab of the spring arm can have an inverted V-shaped profile defining a space in between the tab to receive the sharp distal tip.

The needle can have a change in profile proximal of the sharp distal tip, the change in profile being larger than the opening of the proximal wall of the second portion.

Yet another aspect of the present disclosure includes a safety IV catheter assembly that includes a catheter hub, a catheter tube extending distally from the catheter hub, a needle hub proximal of the catheter hub, a needle extending distally from the needle hub and through the catheter hub and the catheter tube, and including a sharp distal tip, a retaining clip, and a tip protector assembly located on the needle between the catheter hub and the needle hub. The tip protector assembly includes a proximal wall, an opening in the proximal wall accommodating the needle, first and second fingers extending distally from the proximal wall, and a retaining arm extending distally from the proximal wall. The retaining clip extends around distal portions of the first and second fingers. The first finger includes a tab extending radially inward and bearing against the needle to deflect the first finger radially outward against a spring restoring force in a ready to use position. The retaining arm includes a first retaining feature engaging a second retaining feature on the catheter hub to releasably secure the tip protector assembly to the catheter hub. The retaining clip increases the spring restoring force tending to press the first and second fingers radially inward.

The retaining clip can include a pair of flanges extending radially inward and toward one another at a proximal end of the spring clip. The flanges can seat within channels in outer surfaces of the distal portions of the first finger and the second finger to prevent the spring clip from sliding off the distal portions in the ready to use position.

The retaining clip can have an opening accommodating the needle and the first finger at the ready to use position.

The first retaining feature can include a protrusion extending radially inward from the retaining arm. The second retaining feature can include a groove to receive the protrusion.

The first finger can further includes a recess to increase the flexibility of the first finger.

The needle can have a change in profile proximal of the sharp distal tip. The change in profile can be larger than the opening of the proximal wall of the second portion.

Still yet another aspect of the present disclosure includes a safety needle assembly that includes a needle having a sharp distal tip and a needle tip protector mounted on the needle. The needle tip protector includes a base portion defining an opening receiving the needle, first and second fingers extending distally from the base portion, and a resilient member extending between the first and second fingers. The first finger includes a tab extending radially inward and bearing against the needle to deflect the first finger radially outward against a spring restoring force in a ready to use position. Each of the first and second fingers including a transverse opening. Opposite ends of the resilient member are disposed within the transverse openings in the first finger and the second finger. The resilient member exerts a restoring force on the first finger and the second finger when the first finger and the second finger are moved radially outward with respect to each other.

The needle can have a change in profile proximal of the sharp distal tip, the change in profile being larger than the opening of the base portion.

The resilient member can have locking tabs at the opposite ends of the resilient member to retain the opposite ends of the resilient member in the transverse openings of the first finger and the second finger.

The resilient member can have a hole to receive the needle.

The first finger and the second finger can each define a shoulder configured to engage with a catheter hub to retain the needle tip protector to the catheter hub in the ready to use position, and disengage with the catheter hub when the sharp distal tip is received in the needle tip protector.

Other aspects of the present disclosure include a safety IV catheter assembly that includes a catheter hub, a catheter tube extending distally from the catheter hub, a needle hub proximal of the catheter hub, a needle extending distally from the needle hub and through the catheter hub and the catheter tube, the needle including a sharp distal tip, and a tip protector assembly located on the needle between the catheter hub and the needle hub. The tip protector assembly includes a proximal wall, an opening in the proximal wall accommodating the needle, first and second fingers extending distally from the proximal wall, at least one tension strip, a retaining clip, and a retaining arm extending distally from the proximal wall. Opposite ends of the tension strip engages, respectively, the first finger and the second finger to bias the first finger and the second finger toward one another when the first finger and the second finger are moved apart radially with respect to each other. The retaining clip extends around distal portions of the first and second fingers. The first finger includes a tab extending radially inward and bearing against the needle to deflect the first finger radially outward against a spring restoring force. The retaining arm includes a first retaining feature engaging a second retaining feature on the catheter hub to releasably secure the tip protector assembly to the catheter hub.

The retaining clip can include a pair of flanges extending radially inward and toward one another at a proximal end of the spring clip. The flanges can seat within channels in outer surfaces of the distal portions of the first finger and the second finger to prevent the spring clip from sliding off the distal portions in the ready to use position.

The retaining clip can have an opening accommodating the needle and the first finger at the ready to use position.

The first retaining feature can include a protrusion extending radially inward from the retaining arm.

The second retaining feature can comprise a groove to receive the protrusion.

The needle can have a change in profile proximal of the sharp distal tip, the change in profile being larger than the opening of the proximal wall of the second portion.

Another aspect of the present disclosure includes a method of manufacturing a safety needle assembly that includes providing a needle having a sharp distal tip and a needle tip protector comprising a first portion having a channel receiving the needle and a second portion attached to the first portion, and mounting the needle tip protector on the needle. The second portion can have a proximal wall, an opening in the proximal wall receiving the needle, a sidewall extending distally from the proximal wall, first and second spring arms extending transversely from opposite edges of the sidewall, and a third spring arm extending distally from the sidewall. The first spring arm and the second spring arm can extend at least partially around the first portion, and the third spring arm can include a tab extending radially inward and configured to bear against the needle to deflect the third spring arm radially outward against a spring restoring force in a ready to use position.

The first spring arm and the second spring arm can be arcuate. The first spring arm and the second spring arm can be deflected outwardly by the first portion against a spring restoring force to secure the second portion to the first portion.

The first portion can have a mating surface having a depth configured to receive the first spring arm and the second spring arm.

The tab of the third spring arm can have an inverted V-shaped profile defining a space in between the tab to receive the sharp distal tip.

The needle can have a change in profile proximal of the sharp distal tip, the change in profile being larger than the opening of the proximal wall of the second portion.

The first portion and the second portion can each define a shoulder configured to engage with a catheter hub to retain the needle tip protector to the catheter hub in the ready to use position, and disengage with the catheter hub when the third spring arm and the tab elastically return to cover the sharp distal tip.

The second portion can further include a strengthening rib at a junction of the proximal wall and the sidewall for increasing the spring restoring force of the third spring arm.

A still yet further aspect of the present disclosure is a safety needle assembly, comprising: a needle attached to a needle hub, said needle having a sharp distal tip; and a needle tip protector mounted on the needle, the needle tip protector comprising a first portion having a channel receiving the needle and a second portion made from a relatively more rigid material than the first portion attached to the first portion, the second portion having a proximal wall, an opening in the proximal wall receiving the needle, a sidewall extending distally from the proximal wall, , and a distal wall extending radially from the sidewall and abutted by a side of the needle to deflect the sidewall radially outward against a spring restoring force in a ready to use position.

The safety needle assembly can further comprise a first spring arm and a second spring arm extending transversely from opposite edges of the sidewall and partially around the first portion.

The safety needle assembly wherein the first spring arm and the second spring arm can be arcuate and deflected outwardly by the first portion against a spring restoring force to secure the second portion to the first portion.

The safety needle assembly wherein the first portion can have a mating surface having a depth configured to receive the first spring arm and the second spring arm.

The safety needle assembly can further comprise an aperture located next to the opening on the proximal wall for receiving a projection on the first portion.

The safety needle assembly can further comprise a retaining arm extending from the proximal wall, said retaining arm extending axially along a lengthwise axis of the needle and on a side closer to the first portion than the second portion.

The safety needle assembly can further comprise a catheter tube attached to a catheter hub and wherein the needle tip protector can be located in an interior cavity of the catheter hub.

The safety needle assembly can further comprise a catheter tube attached to a catheter hub and wherein the needle tip protector can be located in an interior cavity of the catheter hub and the retaining arm can be located externally of the catheter hub and contacts at least a section of an exterior surface of the catheter hub.

The safety needle assembly wherein the needle can comprise a change in profile for engaging a perimeter on the proximal wall of the second portion.

Yet another aspect of the present disclosure is a method of manufacturing a safety needle assembly. The method can comprise: providing a needle having a lengthwise axis, and a sharp distal tip and connecting the needle to a needle hub having an interior cavity; forming a needle tip protector comprising a first portion having a channel receiving the needle and a second portion made from a relatively more rigid material than the first portion attached to the first portion, the second portion having a proximal wall, an opening in the proximal wall receiving the needle, a sidewall extending distally from the proximal wall, and a distal wall extending radially from the sidewall and abutted by a side of the needle; and mounting the needle tip protector on the needle.

The method can further comprise a first spring arm and a second spring arm extending transversely from opposite edges of the sidewall and partially around the first portion; and wherein the first spring arm and the second spring arm can be arcuate and deflected outwardly by the first portion against a spring restoring force to secure the second portion to the first portion.

The method wherein the first portion can have a lower section with a channel for receiving the sidewall of the second portion.

The method wherein the needle tip protector can further comprise a retaining arm extending from the proximal wall, said retaining arm can extend axially along a lengthwise axis of the needle and on a side closer to the first portion than the second portion.

The method can further comprise a catheter tube attached to a catheter hub and the needle projecting through the catheter tube; and wherein the needle tip protector can be located in an interior cavity of the catheter hub.

The method can further comprise a catheter tube attached to a catheter hub and the needle projecting through the catheter tube; and wherein the needle tip protector can be located in an interior cavity of the catheter hub and the retaining arm can be located externally of the catheter hub and contacts at least a section of an exterior surface of the catheter hub.

The method wherein the needle can comprise a change in profile for engaging a perimeter on the proximal wall of the second portion.

A still yet further aspect of the present disclosure is a safety IV catheter assembly, comprising: a catheter hub with a catheter tube extending distally from the catheter hub; a needle hub proximal of the catheter hub having a needle extending distally from the needle hub and through the catheter hub and the catheter tube, the needle including a sharp distal tip extending distally of a distal end of the catheter tube; and a tip protector assembly located on the needle between the catheter hub and the needle hub or completely inside the catheter hub, the tip protector assembly including: a proximal wall, an opening in the proximal wall accommodating the needle, first and second fingers extending distally from the proximal wall; and a retaining clip extending around distal portions of the first and second fingers or a resilient member extending through both the first and second fingers; wherein the first finger includes a tab extending radially inward and bearing against the needle to deflect the first finger radially outward against a spring restoring force of the retaining clip or the resilient member in a ready to use position; and wherein the retaining clip or the resilient member increases the spring restoring force tending to press the first and second fingers radially inward.

The safety IV catheter assembly wherein the retaining clip can have an opening for accommodating the needle and wherein the tip protector can further comprise an elastic strip for biasing the first and second fingers inwardly towards the needle.

The safety IV catheter assembly wherein the needle can comprise a change in profile proximal of the sharp distal tip for engaging the proximal wall.

The safety IV catheter assembly can further comprise a sleeve or a washer for strengthening the opening on the proximal wall.

### BRIEF DESCRIPTION OF THE DRAWINGS

The various embodiments of the present safety needle assemblies and needle tip protectors now will be discussed in detail with an emphasis on highlighting the advantageous features. These embodiments depict the novel and non-obvious safety needle assemblies and needle tip protectors shown in the accompanying drawings, which are for illustrative purposes only. These drawings include the following figures, in which like numerals indicate like parts:
Figure 1 is a side perspective view of one embodiment of a safety IV needle assembly including a needle tip protector;
Figure 2 is an exploded side perspective view of the needle tip protector of Figure 1;
Figure 3 is an assembled side perspective view of the needle tip protector of Figure 2;
Figure 4 is a side cross-sectional view of the needle tip protector of Figure 1 disposed about the distal tip of the needle;
Figure 5 is a side elevation view of another embodiment of a safety IV needle assembly including a needle tip protector;
Figure 6 is a side perspective view of the needle tip protector of Figure 5 disposed about the distal tip of the needle;
Figure 7 is a side perspective view of the needle tip protector of Figure 6;
Figure 8 is a side elevation view of the needle tip protector of Figure 6;
Figure 9A is a side cross-sectional view of the needle tip protector of Figure 6;
Figure 9B is a perspective view of a spring clip of the needle tip protector assembly.
Figure 10 is a side elevation view of another embodiment of a needle tip protector;
Figure 11 is a side perspective view of the needle tip protector of Figure 10;
Figure 12 is a side cross-sectional view of the needle tip protector of Figure 10;
Figure 13 is a side cross-sectional view of another embodiment of a needle tip protector;
Figure 14 is a side perspective view of the needle tip protector of Figure 13; and
Figure 15 is a side elevation view of another embodiment of a needle tip protector.

### DETAILED DESCRIPTION

The following detailed description describes the present embodiments with reference to the drawings. In the drawings, reference numbers label elements of the present embodiments. These reference numbers are reproduced below in connection with the discussion of the corresponding drawing features.

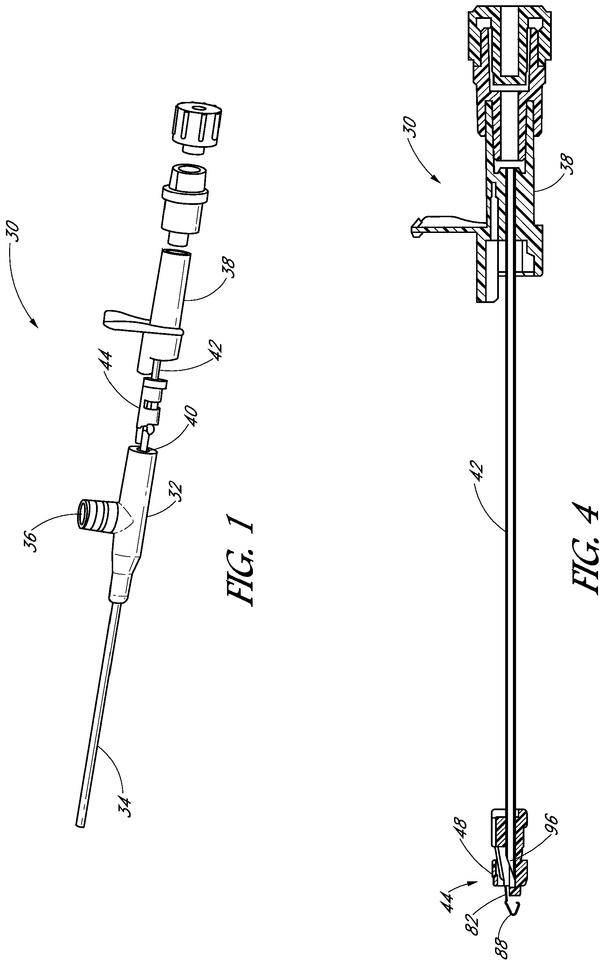

Figure 1 illustrates an IV catheter assembly 30 in accordance with the present embodiments. The catheter assembly 30 includes a catheter hub 32 having a catheter or catheter tube 34 extending distally therefrom. The illustrated catheter hub 32 includes a side port 36, but alternative embodiments may omit the side port 36. A needle hub 38 engages a proximal end 40 of the catheter hub 32. A needle 42 extends distally from the needle hub 38, through the catheter hub 32 and through the catheter tube 34 in a ready to use position, such as ready for performing vascular access. The needle tip of the needle 42 also typically extends distal of the catheter tube opening in the ready position. A needle tip protector 44 is slidably disposed on the needle 42 between the needle hub 38 and the catheter hub 32. In the assembled device, the needle tip protector 44 occupies an interior space of the catheter hub 32. In other embodiments, the needle tip protector 44 is positioned in a space between the catheter hub 32 and the needle hub 38. In yet other embodiments, the catheter hub 32 includes a valve and a valve opener located inside the catheter hub 32 distal of the needle tip protector 44. Exemplary valve and valve opener are disclosed in U.S. Patent Nos. 8,540,728 and 8,591,468, the contents of which are expressly incorporated herein by reference.

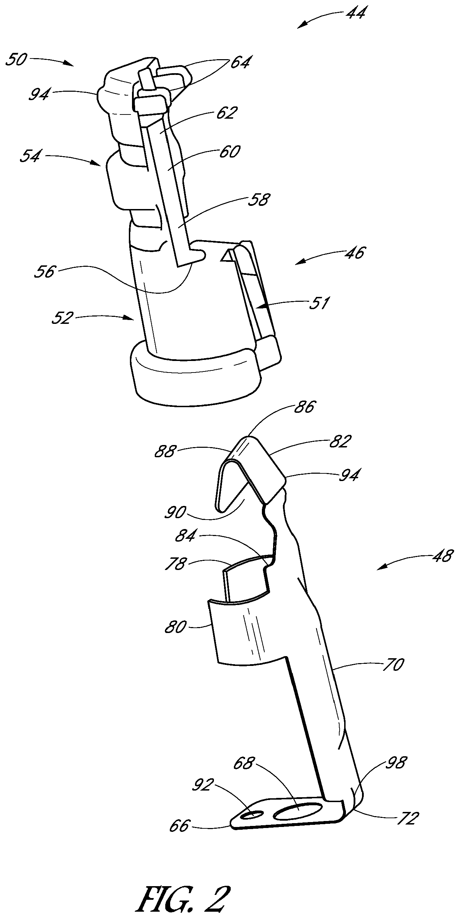

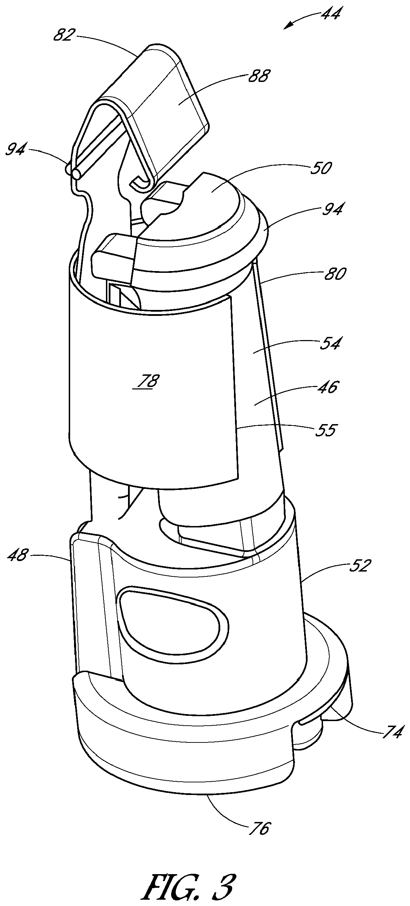

Figures 2 and 3 illustrate the needle tip protector 44 in detail. With reference to Figure 2, the needle tip protector 44 includes a first portion 46 and a second portion 48. With reference to Figure 3, the first portion 46 and the second portion 48 are secured together, as described in further detail below.

With reference to Figure 2, the first portion 46 includes an upper section 50, a lower section 52, and an intermediate section 54 located between the upper and lower sections 50, 52. In the illustrated embodiment, the lower section 52 is substantially cylindrical, and the upper section 50 and the intermediate section 54 are each substantially semi-cylindrical. However, the illustrated shapes should not be interpreted as limiting, and can be any shape. The lower section 52 defines a lower portion 56 of a needle-receiving channel 58, which may be referred to as a first channel. The needle-receiving channel 58, which extends in the direction of a longitudinal axis of the needle tip protector 44, continues through the intermediate section 54 toward and through the upper section 50, and is configured for receiving the needle 42. The lower portion 56 of the needle-receiving channel 58 is a cylindrical channel, while the intermediate portion 60 and the upper portion 62 of the needle-receiving channel 58 are a semi-cylindrical channel. The upper section 50 includes a pair of spaced projections 64 on opposite sides of the needle-receiving channel 58. The projections 64 confines the needle 42 in the needle-receiving channel 58, as the needle 42 is withdrawn from the catheter tube 34 and in a closed position, as further described below.

With continued reference to Figure 2, the second portion 48 includes a proximal wall 66, an opening 68, such as a proximal opening, in the proximal wall 66 that receives the needle 42, and a sidewall 70 extending distally from a first edge 72 of the proximal wall 66. With reference to Figure 3, the proximal wall 66 of the second portion 48 is received within a groove 74 in a lower surface 76 of the lower section 52 of the first portion 46. With reference to Figure 2, first and second spring arms 78, 80 extend transversely from opposite edges of the sidewall 70. The two spring arms 78, 80 may also be referred to as gripping arms. Each of the first and second spring arms 78, 80 is arcuate and extends partially around the upper section 50 and the intermediate section 54 of the first portion 46, as shown in Figure 3. The sidewall 70 is positioned in a second channel 51, defined by two raised ribs, located in the lower section 52 when the second portion attaches to the first portion. The second channel 51 is spaced from the first channel 58. The first and second spring arms 78, 80 thus assist in holding the first and second portions 46, 48 together. In another embodiment, an inner diameter of the second portion 48 at the first and second spring arms 78, 80 is less than or equal to a diameter of the first portion engaging with the first and second spring arms 78, 80. Thus, the first and second spring arms 78, 80 are deflected outwardly, such as being biased outwardly, by the first portion 46 against a spring restoring force, thereby securing the second portion 48 to the first portion 46. Gripping features, such as projections, may be incorporated at the free end of each arm and/or on an interior surface of each arm to facilitate gripping the first portion. Alternatively, the first portion 46 can have a mating surface 55 engaging with the first and second arms 78, 80. The mating surface 55 can have a depth thereby creating a small channel receiving the first and second spring arms 78, 80. The mating surface 55 can also have a width slightly greater than or equal to a width of the first and second spring arms 78, 80 to prevent the second portion 48 from sliding distally or proximally relative to the first portion 46.

With reference to Figure 2, a third spring arm 82 extends distally from a distal edge 84 of the sidewall 70. A distal end 86 of the third spring arm 82 includes a tab 88 defining an inverted V-shape in profile, and extending radially inward from the third spring arm 82. The tab 88 bears against the needle 42 to deflect the third spring arm 82 radially outward against a spring restoring force. The tab 88 may also be referred to as a distal wall for blocking the needle tip in a protective position. Further, the inverted V-shape of the tab 88 defines a space 90 beneath the tab 88 that covers and/or stops the distal tip of the needle 42 once the needle tip is received in the needle tip protector 44, thereby preventing the needle tip from being exposed.

The proximal wall 66 of the second portion 48 further includes an aperture 92 that receives a pin (not shown) in the lower section 52 of the first portion 46 when the second portion 48 is assembled with the first portion 46. In some embodiments, the pin extends through the aperture 92 of the second portion 48 and is deformed by heat and/or pressure in order to increase the diameter of the pin, to create an interference or compression fit. By making the diameter of the pin larger than the diameter of the aperture 92, the second portion 48 is secured to the first portion 46. In alternative embodiments, the second portion 48 may be secured to the first portion 46 by other means, such as welding, gluing, or any other suitable means of connection.

With reference to Figure 1, prior to use of the IV catheter assembly 30, the needle 42 extends through the catheter tube 34 and the sharp needle tip extends out the distal opening of the tube, and the needle tip protector 44 is housed partially or entirely in the catheter hub 32. With reference to Figure 3, the tab 88 of the second portion 48 rests against the needle 42 (not shown in Figure 3), thereby deflecting the third spring arm 82 radially outward away from the needle 42 and creating a spring restoring force in the third spring arm 82 in the ready to use position. The first and second spring arms 78, 80 extend around the first portion 46 and assist in preventing the first and second portions 46, 48 from separating as the third spring arm is deflected outward. The needle 42 is securely positioned in the needle-receiving channel 58 (Figure 2) of the second portion 48.

With reference to Figures 1 and 4, as the needle 42 is withdrawn from the catheter tube 34, the needle 42 moves relative to the needle tip protector 44 until the needle tip 96 is received in the needle tip protector 44. Just proximal of the needle tip 96, the needle 42 includes an enlargement (not shown), which may be referred to as a change in profile and can include a crimp or a bulge. The enlargement has a greater diameter than the opening 68 in the proximal wall 66 of the needle tip protector 44 (Figure 2), and thus cannot pass through the opening 68 in the proximal wall 66. When the enlargement engages the proximal wall 66, such as the perimeter of the opening 68, the needle tip protector 44 is pulled out of the catheter hub 32 together with the needle 42. Just prior to the enlargement engaging the proximal wall 66, the needle tip 96 passes proximally of the tab 88 of the second portion 48, thereby releasing the spring restoring force in the third spring arm 82 and causing the third spring arm 82 to move radially inward to cover the needle tip 96. After the needle tip protector 44 is pulled out of the catheter hub 32, the sharp distal tip of the needle 42 is safely enclosed within the needle tip protector 44 in the closed position, as shown in Figure 4.

Prior to withdrawing the needle 42 from the catheter tube 34, the needle tip protector 44 is retained in the catheter hub 32. In order to retain the needle tip protector 44 in the catheter hub 32, both the upper section 50 of the first portion 46 and the upper section 50 of the second portion 48 include a shoulder 94, as shown in Figures 2 and 3. The shoulders 94 of the first and second potions 46, 48 engage one or more retention features (not shown) provided in the catheter hub 32 to retain the needle tip protector 44 in the catheter hub 32. The retention feature(s) may comprise, for example, projections, protrusions, recesses, depressions, etc., or combinations thereof. For example, the retention feature(s) may comprise an annular ring or groove extending along the entire inner periphery of the catheter hub 32, or one or more ring or groove segments extending along only a respective part of the inner periphery of the catheter hub 32. The shoulders may also be referred to as hub engagement surface while the retention feature may be referred to as a guard engagement surface.

As described above, and with reference to Figure 4, when the needle tip 96 passes proximally of the tab 88 of the second portion 48, the third spring arm 82 moves radially inward to cover the needle tip 96. This movement disengages the shoulders 94 of the needle tip protector 44 from the retention feature(s) on the catheter hub 32, thereby releasing the needle tip protector 44 from the catheter hub 32.

The first and second portions 46, 48 of the needle tip protector 44 may be constructed of any suitable biocompatible material(s), such as one or more polymers, metals, ceramics, etc. In one embodiment, the first portion 46 is constructed of a polymer and the second portion 48 is constructed of a metal. The second portion 48 may, for example, be formed as a single piece from a strip of suitable metal sheet having spring-like properties. Non-limiting examples of polymers from which the first and/or second portions 46, 48 may be constructed include nylon, polyethylene, polyurethane, ethylene-vinyl acetate (EVA), polyether block amide (PEBAX), acrylonitrile butadiene styrene (ABS), polyether ether ketone (PEEK), polytetrafluoroethylene (PTFE), thermoplastic polyetherimide (ULTEM), etc. Non-limiting examples of metals from which the first and/or second portions 46, 48 may be constructed include stainless steel, titanium, cobalt-chromium, etc. Non-limiting examples of ceramics from which the first and/or second portions 46, 48 may be constructed include porcelain, alumina, hydroxyapatite, zirconia, etc. Thus, when the first portion or finger and the second portion or finger are made from different materials, the second portion or finger can be said to be made from a relatively more rigid material than the first portion or finger.

With reference again to Figure 2, the second portion 48 may include a strengthening rib 98 at the junction of the proximal wall 66 and the sidewall 70. The strengthening rib 98 increases the spring restoring force of the third spring arm 82. For example, when assembled over a needle 42 in the ready to use position, the needle 42 biases the third spring arm 82 away from the first portion 46. As the needle tip 96 retracts proximally of the tab 88, the third spring arm 82 recoils, such as moving in the direction of the first portion 46, to cover the needle tip 96. The rib 98 facilitates the inward biasing of the spring arm 82.

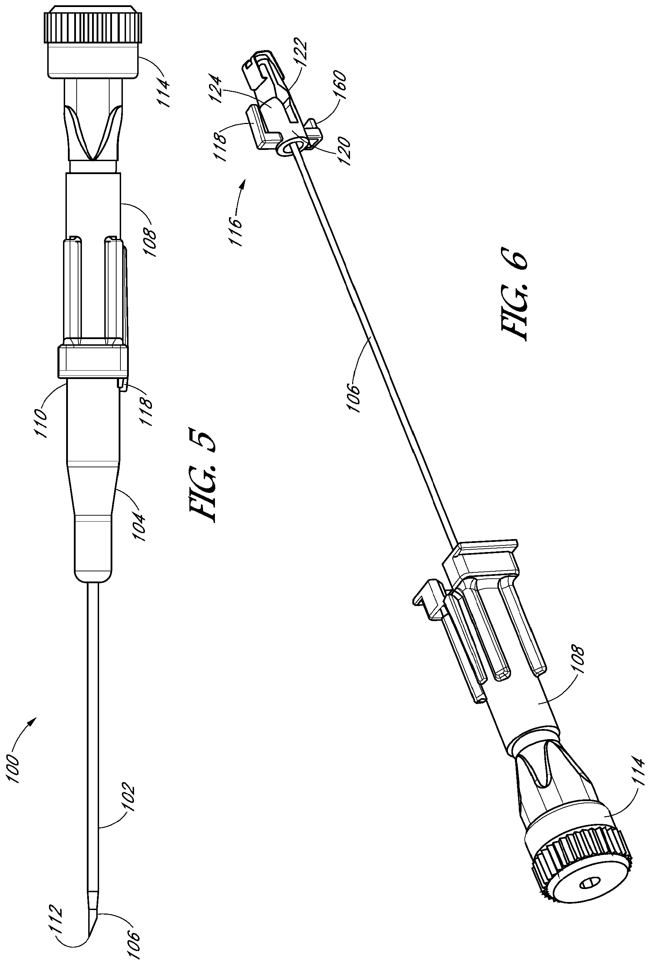

Figure 5 illustrates another embodiment of a catheter assembly 100. The catheter assembly 100 includes a catheter tube 102 extending distally from a catheter hub 104. A needle 106 extends distally from a needle hub 108 through the catheter hub 104 and through the catheter tube 102. The needle hub 108 releasably engages a proximal end 110 of the catheter hub 104. The needle 106 includes a sharp distal tip 112 that protrudes from the catheter tube 102 at the distal end thereof. The needle hub 108 includes a port member 114 connected to the needle hub, such as in a Luer slip arrangement. The port member 114 may be a vent plug having a vent filter located on a proximal end thereof to vent air or gas during primary blood flashback.

The catheter hub 104 defines an inner chamber (not shown) in which a needle tip protector assembly 116 is seated. In Figure 5, only a retaining arm 118 of the needle tip protector assembly 116 is visible. The needle tip protector assembly 116 is slidably arranged on the needle 106, such that it moves along the needle 106 when the needle 106 is withdrawn from the catheter tube 102, as described below. With reference to Figure 6, the needle tip protector assembly 116 covers the sharp distal tip 112 after the needle 106 has been used to prevent accidental needle pricks.

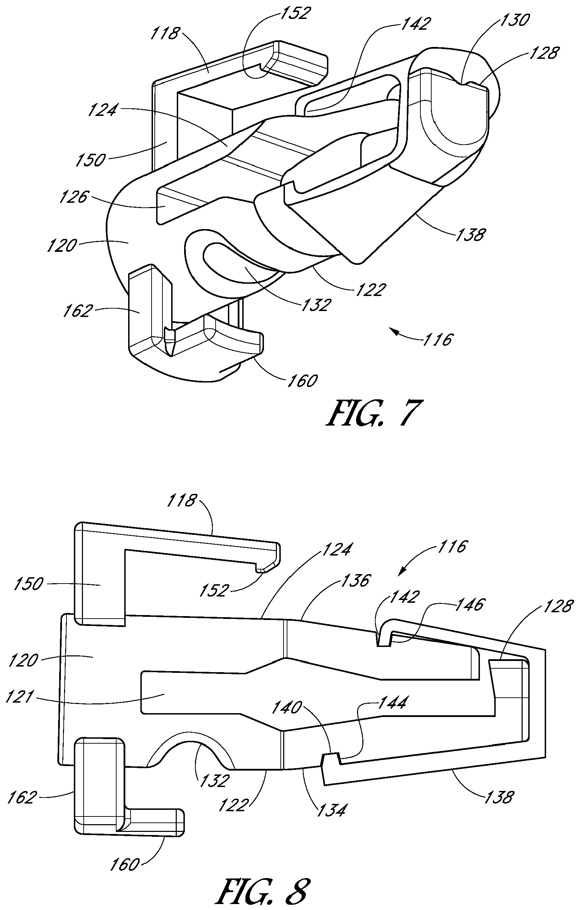

With reference to Figures 7-9, the needle tip protector assembly 116 includes a generally cylindrical base portion 120 at a proximal end of the needle tip protector assembly 116. First and second fingers 122, 124 extend from a distal face 126 of the base portion 120 generally in the axial direction. The first finger 122 is longer than the second finger 124. A tab 128 extends radially inward at a distal end of the first finger 122. The tab 128 may be referred to as a distal wall or a radially extending distal wall. The tab 128 overlaps with the second finger 124, such that when the needle 106 is withdrawn from the catheter tube 102 and the catheter hub 104 the sharp distal tip 112 of the needle 106 is received in the needle tip protector assembly 116 such that the tab 128 blocks the sharp distal tip 112 from protruding distally from the needle tip protector assembly 116 (Figure 6). The tab 128 may have a sufficiently wide wall to retain the needle within the holding space 121 defined by the two fingers 122, 124, the base portion 120 and the tab. For example, if the needle pivots about an opening formed through the base portion 120, the tab is sufficiently wide to prevent the needle tip from deflecting past the side of the tab to be exposed outside the holding space 121.

In the ready to use position, the needle 106 extends through the needle tip protector assembly 116 and the tab 128 bears against the needle 106 to deflect the first finger 122 radially outward and away from the second finger 124. With reference to Figure 7, an inner face (facing the needle 106) of the tab 128 can include an axial groove 130 that supports the needle 106, such as to act as a guide for the needle, to maintain the needle 106 aligned with a longitudinal axis of the needle tip protector assembly 116 as the needle 106 is pulled through the needle tip protector assembly 116 during withdrawal of the needle 106 from the catheter tube 102. With reference to Figures 7-9A, an outer surface of a proximal section of the first finger 122 includes a recess 132 to increase the flexibility of the first finger 122.

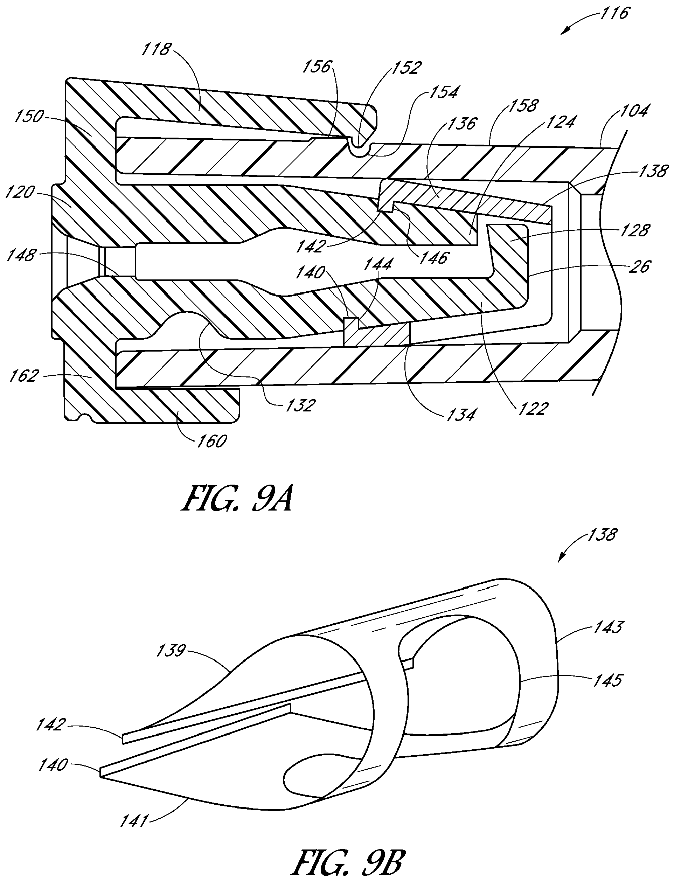

With reference to Figures 8, 9A, and 9B, distal sections 134, 136 of the first and second fingers 122, 124, respectively, have outer surfaces that taper inward toward the distal ends of the first and second fingers 122, 124. A spring clip 138 overlaps the first and second fingers 122, 124 in the region of the distal sections 134, 136. The spring clip 138, which is illustrated in detail in Figures 9A and 9B, includes a pair of flanges 140, 142 that extend radially inward and toward one another at a proximal end of the spring clip 138. For example, the flanges can each be bent from the first and second leaves 139, 141 of the spring clip. The flanges 140, 142 seat within channels 144, 146, respectively, in the outer surfaces of the distal sections 134, 136 of the first and second fingers 122, 124. Engagement of the flanges 140, 142 within the channels 144, 146 prevents the spring clip 138 from sliding off the distal sections 134, 136 toward the proximal end of the needle tip protector assembly 116 or distally off of the two fingers 122, 124 when the first and second fingers 122, 124 are spread apart by the needle 106 extending through the needle tip protector assembly 116.

With reference to the detail view of the spring clip 138 in Figure 9B, the spring clip 138 includes first and second leaves 139, 141 that extend proximally from a bent distal end 143 of the spring clip 138. The spring clip 138 is preferably constructed of a resilient material, such as a polymer or a metal, that stores a spring restoring force when the first and second leaves 139, 141 are spread apart and overlapped with the first and second fingers 122, 124 of the needle tip protector assembly 116, as shown in the cross-sectional view of Figure 9A. The spring clip 138 thus increases the restoring force tending to press the first and second fingers 122, 124 radially inward after the first and second fingers 122, 124 are moved radially outward away from one another by the needle 106 interposed between the first and second fingers 122, 124, and after the needle tip moves proximally of the tab 128. The spring clip 138 further includes an opening 145 at its distal end 143 that extends proximally through a center portion of the second leaf 141. The opening 145 accommodates the needle 106 and the first finger 122 when the first and second fingers 122, 124 are moved radially outward away from one another by the needle 106 interposed between the first and second fingers 122, 124. For example, the first finger 122 may extend out the opening 145 when the tab 128 is pressing against the needle 106 in the ready to use position or prior to the closed position.

With further reference to Figure 9, an axial bore 148 extends through the base portion 120 of the needle tip protector assembly 116 for receiving the needle 106. The needle 106 includes an enlargement (not shown), such as a change in profile, just proximal of the sharp distal tip 112. A diameter of the enlargement is larger than a diameter of the bore 52. Thus, the enlargement prevents the needle tip protector assembly 116 from sliding off the needle 106 when the sharp distal tip 112 is received between the first and second fingers 122, 124 and covered by the needle tip protector assembly 116. In some examples, a rigid sleeve or a washing having an opening is formed with the bore to increase the strength of the bore to prevent the enlargement from deforming and separating from the bore. The sleeve or washer may be made from a hard plastic or from a metal material.

The needle tip protector assembly 116 further includes the retaining arm 118 to secure the needle tip protector assembly 116, or at least the two fingers 122, 124 and the spring clip 138, within the catheter hub 104 and, in particular, to prevent removal of the needle tip protector assembly 116 from the catheter hub 104 during withdrawal of the needle 106 before the sharp distal tip 112 is safely received within the needle tip protector assembly 116. The retaining arm 118 extends distally from a proximal portion of the needle guard, such as from the base portion 120 of the needle tip protector assembly 116, via a transverse segment 150. The transverse segment 150 extends radially outwardly from the base portion 120 on the same side as the second finger 124. The retaining arm 118 extends distally from the transverse segment 150 and is slightly angled toward the second finger 124. In some embodiments, the retaining arm 118 forms an angle in the range of about 0°-10° with the longitudinal axis of the needle tip protector assembly 116, such that a clearance between the retaining arm 118 and the second finger 124 narrows toward the distal end of the retaining arm 118.

A hook-like protrusion 152 extends radially inward at the distal end of the retaining arm 118. The protrusion 152 engages a groove or recess 154 located just distal of a protrusion 156 on the outer surface 158 of the catheter hub 104. The engagement of the protrusion 152 of the retaining arm 118 with the combination of the protrusion 156 and the recess 154 on the catheter hub 104 prevents the needle tip protector assembly 116 from moving axially relative to the catheter hub 104. The needle tip protector assembly 116 stays engaged with the catheter hub 104 until a pulling force exerted by the needle 106 on the base portion 120 of the needle tip protector assembly 116 via the enlargement of the needle 106 upon withdrawal of the needle 106 from the catheter tube 102 becomes great enough to disengage the retaining arm 118 from the protrusion 156 of the catheter hub 104.

A supporting arm 160 extends distally from a transverse segment 162. The transverse segment 162 extends radially outward from the base portion 120 in a direction opposite the transverse segment 150. The supporting arm 160 forms a generally right angle with the transverse segment 162. The supporting arm 160 is configured to be in contact with the outer surface 158 of the catheter hub 104 across substantially its entire length and width. The supporting arm 160 thus prevents the needle tip protector assembly 116 from moving in a radial direction within the catheter hub 104. The supporting arm 160 may also help to secure the needle tip protector assembly 116 against axial movement with respect to the catheter hub 104, thereby adding to the securement of the needle tip protector assembly 116 within the catheter hub 104. However, the supporting arm 160 does not necessarily have to be in such a retaining relationship with the catheter hub 104. In the illustrated embodiment, the retaining arm 118 is provided on the side of the base portion 120 adjacent the second finger 124, and the supporting arm 160 is provided on the side of the base portion 120 adjacent the first finger 122. However, this configuration may be reversed in other embodiments. As shown, the retaining arm 118 is longer than the supporting arm 160, such as being about 1.8 to 3 times longer than the supporting arm to provide adequate retaining and supporting balance.

In the illustrated embodiment, the base portion 120, the first and second fingers 122, 124, the retaining arm 118, and the supporting arm 160, are integrally formed. For example, these components may be formed from a polymer material via injection molding or a combination of a polymer or thermoplastic material via co-molding, over molding or insert molding. However, in alternative embodiments some or all of the foregoing components may be made as separate pieces later secured to one another, for example, by gluing, welding, soldering, or the like. Further one or more of the foregoing components may be made of a material that is different from the material of the other components, such as plastic and elastomer.

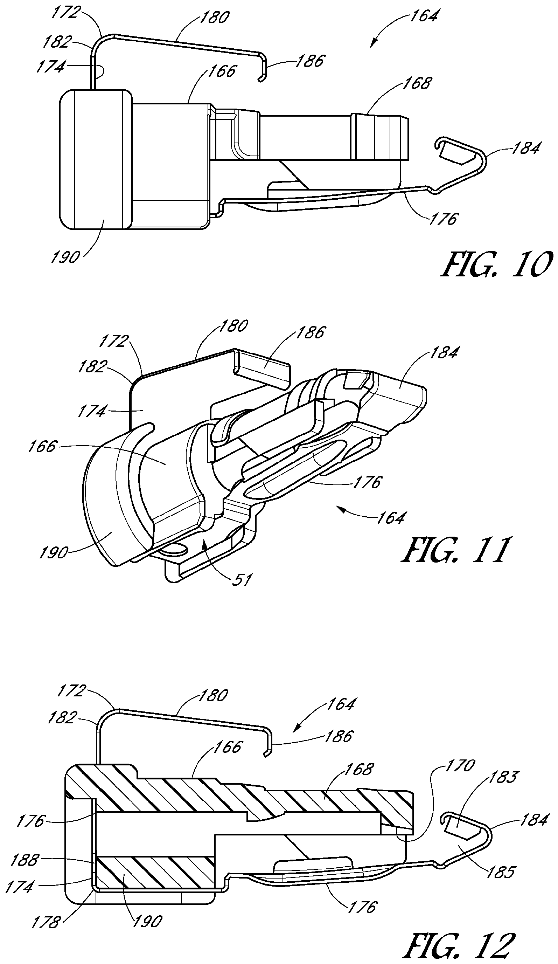

Figures 10-12 illustrate another embodiment of a needle tip protector assembly 164 that is configured to be used in the catheter assembly 100 described above and shown in Figure 5. The needle tip protector assembly 164 is similar to the needle tip protector assembly 116 described above, but does not include a supporting arm or a first finger that is integrally formed with the base portion 120, the second finger 124, and the retaining arm 118.

Instead, the needle tip protector assembly 164 includes a first portion 166 having a first finger 168 with a channel 170 (Figure 12) for receiving the needle 106 and a second portion 172. The channel 170 for receiving the needle may be referred to as a first channel. With reference to Figure 12, the second portion 172 includes a proximal wall 174, an opening 176 in the proximal wall 174 for accommodating the needle 106, a spring arm or second finger 176 extending distally from a first edge 178 of the proximal wall 174, and a retaining arm 180 extending distally from a second edge 182 of the proximal wall 174. Part of the spring arm or spring finger 176 is positioned in a channel 51 on the base portion 190 of the first portion. The channel 51 is defined by two spaced apart raised ribs, as more clearly shown in FIG. 11, and may be referred to as a second channel, which is spaced from the first channel. A distal end of the second finger 176 includes a tab 184 extending radially inward and bearing against the needle 106 to deflect the second finger 176 radially outward against a spring restoring force. The tab 184 can define an inverted V-shape in profile defining a space 185 beneath the tab 88 that covers and/or stops the distal tip of the needle 106 once the needle tip 112 is received in the needle tip protector assembly 164, thereby preventing the needle tip 112 from being exposed. A pair of bent portions 183 can extend inwardly from the tab 184 to maintain the needle tip 112 in the needle tip protector assembly 164.

A distal end of the retaining arm 180 includes a protrusion 186 extending radially inward and configured to engage a groove or recess on the catheter hub (not shown) to releasably secure the needle tip protector assembly 164 to the catheter hub.

In the embodiment of Figures 10-12, the second portion 172 may be, for example, a unitary strip of sheet metal that is bent to form the proximal wall 174, the first finger 176, the tab 184, the retaining arm 180, and the protrusion 186. The second portion 172 may be secured to the first portion 166 by any suitable means, such as gluing, welding, etc. For example, the proximal wall 174 may be glued or welded to a proximal face 188 (Figure 12) of the base portion 190. As described above with respect to the previous embodiment, distal sections of the first and second fingers 168, 176 are surrounded by the spring clip 138 shown in Figure 9A (not shown in Figures 10-12 for clarity), such that the first and second fingers 168, 176 can be spread apart against a restoring force of the spring clip 138.

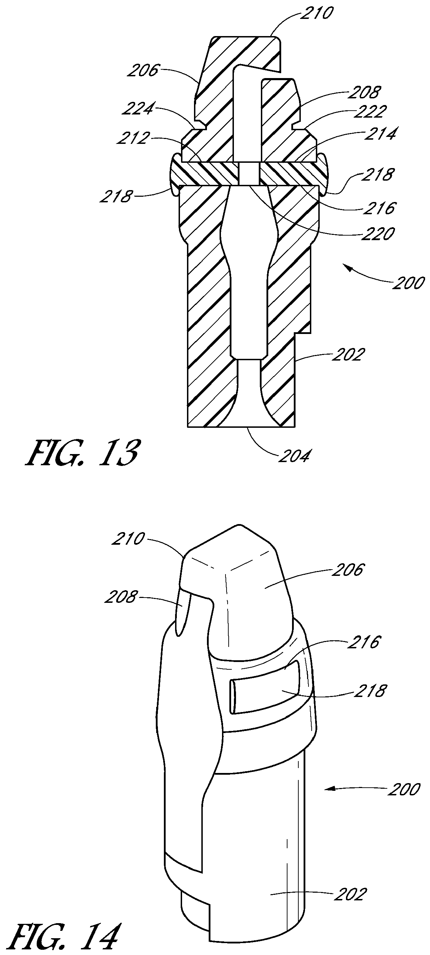

Figures 13 and 14 illustrate another embodiment of a needle tip protector 200 configured for use with an IV catheter assembly, such as the IV catheter assembly 30 described above and illustrated in Figure 1 or the IV catheter assembly 100 described above and illustrated in Figure 5. With reference to Figure 13, the needle tip protector 200 comprises a base portion 202 defining an opening 204 for receiving the needle 42, 106. The opening 204 has a diameter that is smaller than the diameter of an enlargement near the distal tip 96, 112 of the needle 42, 106, such that the needle 42, 106 cannot be pulled through the opening 204. Thus, as the needle 42, 106 is withdrawn from the catheter tube 34, 102, the sharp distal tip 96, 112 becomes entrained within the needle tip protector 200 in a manner similar to that described above with respect to the previous embodiments.

First and second fingers 206, 208 extend distally from the base portion 202. The first finger 206 is longer than the second finger 208, and includes a tab 210 extending radially inward at the distal end of the first finger 206 and overlapping the distal end of the second finger 208. The tab 210 bears against the needle to deflect the first finger 206 radially outward against a spring restoring force, similarly to that described above with respect to the foregoing embodiments. Once the needle tip is disposed within the needle tip protector 200, the tab 210 overlaps the distal tip 96, 112 to prevent the distal tip 96, 112 from protruding distally outward of the needle tip protector 200, similarly to that described above with respect to the foregoing embodiments.

Each of the first and second fingers 206, 208 includes a transverse opening 212, 214. A resilient member 216 extends between the first and second fingers 206, 208. Opposite ends of the resilient member 216 are received within the transverse openings 212, 214. When the first and second fingers 206, 208 are moved radially outward with respect to each other by the needle 42, 106 being interposed between the first and second fingers 206, 208, the resilient member 216 stretches and exerts a restoring force on the first and second fingers 206, 208. The resilient member 216 includes a locking tab 218 at opposite ends, each locking tab 218 having a greater outer dimension than the inner dimensions of the transverse openings 212, 214. The locking tabs 218 thus retain the opposite ends of the resilient member 216 in the transverse openings 212, 214 when the first and second fingers 206, 208 are moved radially outward with respect to each other. The resilient member 216 includes an opening 220 that is aligned with the opening 204 in the base portion 202. The opening 220 accommodates the needle 42, 106 prior to the needle 42, 106 being withdrawn from the needle tip protector 200. As soon as the sharp distal tip 96, 112 of the needle 42, 106 enters the needle tip protector 200 and as the needle 42, 106 is withdrawn from the catheter assembly, the first finger 206 snaps back into its unstressed position (as shown in Figure 13) due to the restoring force exerted by the resilient member 216 on the first and second fingers 206, 208.

The outer surface of each finger 206, 208 includes a locking shoulder 222 that forms a recess or groove 224. An inner surface of the catheter hub includes a complementary locking feature or surface, such as an annular locking projection. As the needle 42, 106 is withdrawn through the catheter hub, the locking features on the catheter hub and the needle tip protector 200 disengage to release the needle tip protector 200 from the catheter hub, similarly to that described above with respect to the foregoing embodiments.

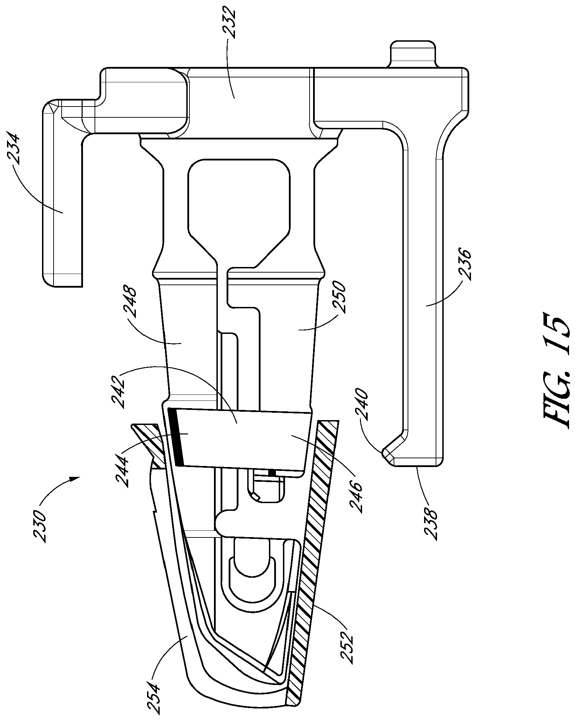

Figure 15 illustrates another embodiment of a needle tip protector 230 configured for use with an IV catheter assembly, such as the IV catheter assembly 30 described above and illustrated in Figure 1 or the IV catheter assembly 100 described above and illustrated in Figure 5. The needle tip protector 230 comprises a base portion 232 with first and second arms 234, 236 extending distally from opposite ends of the base portion 232. The first arm 29 has a shorter length than the second arm 236. A distal end 238 of the second arm 236 includes a locking protrusion 240 that extends radially inward to engage a corresponding recess or projection on the outer surface of the catheter hub (not shown). The locking protrusion 240 holds the needle tip protector 230 in position as the needle 42, 106 is withdrawn in similar fashion as described above with respect to the foregoing embodiments. When the enlargement on the needle contacts the base portion 232, the needle tip protector 230 disengages the catheter hub in similar fashion as described above with respect to the foregoing embodiments.

The embodiment of Figure 15 further includes two tension strips 242 (only one is visible in Figure 15; the other is on the opposite side). Opposite ends 244, 246 of each tension strip 242 are fixed to first and second fingers 248, 250 for biasing the fingers 248, 250 radially inward toward each other. The fingers 248, 250 extend distally from the base portion 232 at a position located radially inward from the first and second arms 234, 236. In some embodiments, the ends of the tension strips 242 may be co-molded with their respective fingers 248, 250. In other embodiments, the ends 244, 246 of the tension strips 242 may be secured to their respective fingers 248, 250 by any suitable means, such as adhesive and/or a mechanical (tab and slot) engagement.

The embodiment of Figure 15 further includes a spring clip 252. The spring clip 252, which is similar in structure and function to the spring clip 138 described above and illustrated in Figure 9, overlaps the first and second fingers 248, 250 of the needle tip protector 230 to increase the restoring force tending to pull the first and second fingers 248, 250 radially inward after the first and second fingers 248, 250 are moved radially outward away from one another by the needle interposed between the first and second fingers 248, 250. The spring clip 252 further includes an opening 254 at its distal end that accommodates the needle and the first finger 248 when the first and second fingers 1248, 250 are moved radially outward away from one another by the needle interposed between the first and second fingers 248, 250.

Methods of making and of using the needle assemblies and their components described herein are contemplated and are considered within the scope of the present disclosure.

The above description presents various embodiments of the present invention, and the manner and process of making and using them, in such full, clear, concise, and exact terms as to enable any person skilled in the art to which it pertains to make and use this invention. This invention is, however, susceptible to modifications and alternate constructions from that discussed above that are fully equivalent. Consequently, this invention is not limited to the particular embodiments disclosed. On the contrary, this invention covers all modifications and alternate constructions coming within the spirit and scope of the invention as generally expressed by the following aspects and claims, which particularly point out and distinctly claim the subject matter of the invention.

### ASPECTS:

1. A safety needle assembly, comprising:
   a needle attached to a needle hub, said needle having a sharp distal tip; and
   a needle tip protector mounted on the needle, the needle tip protector comprising a first portion having a first channel receiving the needle and a second portion made from a relatively more rigid material than the first portion attached to a second channel of the first portion, the second portion having a proximal wall, an opening in the proximal wall receiving the needle, a sidewall extending distally from the proximal wall, and a distal wall extending radially from the sidewall and abutted by a side of the needle to deflect the sidewall radially outward against a spring restoring force in a ready to use position.
2. The safety needle assembly of aspect 1, further comprising a first spring arm and a second spring arm extending transversely from opposite edges of the sidewall and partially around the first portion.
3. The safety needle assembly of aspect 2, wherein the first spring arm and the second spring arm are arcuate and are deflected outwardly by the first portion against a spring restoring force to secure the second portion to the first portion.
4. The safety needle assembly of any one of the preceding aspects, wherein the first portion has a mating surface having a depth configured to receive the first spring arm and the second spring arm.
5. The safety needle assembly of any one of the preceding aspects, further comprising an aperture located next to the opening on the proximal wall for receiving a projection on the first portion.
6. The safety needle assembly of any one of the preceding aspects, further comprising a retaining arm extending from the proximal wall, said retaining arm extending axially along a lengthwise axis of the needle and on a side closer to the first portion than the second portion.
7. The safety needle assembly of any one of the preceding aspects, further comprising a catheter tube attached to a catheter hub and wherein the needle tip protector is located in an interior cavity of the catheter hub.
8. The safety needle assembly of any one of aspects 1-6, further comprising a catheter tube attached to a catheter hub and wherein the needle tip protector is located in an interior cavity of the catheter hub and the retaining arm is located externally of the catheter hub and contacts at least a section of an exterior surface of the catheter hub.
9. The safety needle assembly of any one of the preceding aspects, wherein the needle comprises a change in profile for engaging a perimeter on the proximal wall of the second portion.
10. A method of manufacturing a safety needle assembly, comprising:
   providing a needle having a lengthwise axis, and a sharp distal tip and connecting the needle to a needle hub having an interior cavity;
   forming a needle tip protector comprising a first portion having a first channel receiving the needle and a second portion made from a relatively more rigid material than the first portion attached to a second channel of the first portion, the second portion having a proximal wall, an opening in the proximal wall receiving the needle, a sidewall extending distally from the proximal wall, and a distal wall extending radially from the sidewall and abutted by a side of the needle; and
   mounting the needle tip protector on the needle.
11. The method of aspect 10, further comprising a first spring arm and a second spring arm extending transversely from opposite edges of the sidewall and partially around the first portion; and wherein the first spring arm and the second spring arm are arcuate and are deflected outwardly by the first portion against a spring restoring force to secure the second portion to the first portion.
12. The method of aspect 10 or 11, wherein the first portion has a lower section with a channel for receiving the sidewall of the second portion.
13. The method of any one of aspects 10-12, wherein the needle tip protector further comprises a retaining arm extending from the proximal wall, said retaining arm extending axially along a lengthwise axis of the needle and on a side closer to the first portion than the second portion.
14. The method of any one of aspects 10-13, further comprising a catheter tube attached to a catheter hub and the needle projecting through the catheter tube; wherein the needle tip protector is located in an interior cavity of the catheter hub.
15. The method of any one of aspects 10-13, further comprising a catheter tube attached to a catheter hub and the needle projecting through the catheter tube; wherein the needle tip protector is located in an interior cavity of the catheter hub and the retaining arm is located externally of the catheter hub and contacts at least a section of an exterior surface of the catheter hub.
16. The method of any one of aspects 10-15, wherein the needle comprises a change in profile for engaging a perimeter on the proximal wall of the second portion.
17. A safety IV catheter assembly, comprising:
   a catheter hub with a catheter tube extending distally from the catheter hub;
   a needle hub proximal of the catheter hub having a needle extending distally from the needle hub and through the catheter hub and the catheter tube, the needle including a sharp distal tip extending distally of a distal end of the catheter tube; and
   a tip protector assembly located on the needle between the catheter hub and the needle hub or completely inside the catheter hub, the tip protector assembly including:
      a proximal wall, an opening in the proximal wall accommodating the needle, first and second fingers extending distally from the proximal wall; and
      a retaining clip extending around distal portions of the first and second fingers or a resilient member extending through both the first and second fingers;
      wherein the first finger includes a tab extending radially inward and bearing against the needle to deflect the first finger radially outward against a spring restoring force of the retaining clip or the resilient member in a ready to use position; and
      wherein the retaining clip or the resilient member increases the spring restoring force tending to press the first and second fingers radially inward.
18. The safety IV catheter assembly of claim 17, wherein the retaining clip has an opening for accommodating the needle and wherein the tip protector further comprises an elastic strip for biasing the first and second fingers inwardly towards the needle.
19. The safety IV catheter assembly of any one of aspects 17 and 18, wherein the needle comprises a change in profile proximal of the sharp distal tip for engaging the proximal wall.
20. The safety IV catheter assembly of any one of aspects 17 to 19, further comprising a sleeve or a washer for strengthening the opening on the proximal wall.

## Claims

1. A safety IV catheter assembly (100), comprising:
a catheter hub (32) with a catheter tube (34) extending distally from the catheter hub (32);
a needle hub (38) proximal of the catheter hub (32) having a needle (42, 106) extending distally from the needle hub (38) and through the catheter hub (32) and the catheter tube (34), the needle (42, 106) including a sharp distal tip extending distally of a distal end of the catheter tube (34); and
a tip protector assembly (164, 200) located on the needle (42, 106) between the catheter hub (32) and the needle hub (38) or completely inside the catheter hub (32), the tip protector assembly (164, 200) including:
a proximal wall (174), an opening (176) in the proximal wall (174) accommodating the needle (42, 106), first and second fingers (168, 176, 206, 208) extending distally from the proximal wall (174); and
a retaining clip (138) extending around distal portions of the first and second fingers (168, 176) or a resilient member (216) extending through both the first and second fingers (206, 208);
wherein the first finger (168, 206) includes a tab (184, 210) extending radially inward and bearing against the needle (42, 106) to deflect the first finger (168, 206) radially outward against a spring restoring force of the retaining clip (138) or the resilient member (216) in a ready to use position; and
wherein the retaining clip (138) or the resilient member (216) increases the spring restoring force tending to press the first and second fingers radially inward.

2. The safety IV catheter assembly (100) of claim 1, wherein the retaining clip (138) has an opening (145) for accommodating the needle (42, 106) and wherein the tip protector (164, 200) further comprises an elastic strip for biasing the first and second fingers (168, 176, 206, 208) inwardly towards the needle (42).

3. The safety IV catheter assembly (100) of any one of claims 1 and 2, wherein the needle (42, 106) comprises a change in profile proximal of the sharp distal tip for engaging the proximal wall (174).

4. The safety IV catheter assembly (100) of any one of claims 1 to 3, further comprising a sleeve or a washer for strengthening the opening (176) on the proximal wall (174).

5. The safety IV catheter assembly (100) of any one of claims 1 to 4, further comprising a retaining arm (118) extending from the proximal wall (66), said retaining arm (118) extending axially along a lengthwise axis of the needle (42) and on a side closer to the first portion (46) than the second portion (48).
